# EUROPÄISCHE PATENTSCHRIFT

(11) **EP 0 412 052 B1**
(45) Veröffentlichungstag und Bekanntmachung des Hinweises auf die Patenterteilung: **12.05.1993**
(21) Anmeldenummer: 90810550.5
(22) Anmeldetag: 18.07.1990
(51) Int. Cl.: A61F 5/01

(54) **Orthese zur Behandlung von Bänderrissen oder -zerrungen am lateralen Fussknöchel**
Orthosis for medical treatment of ligament ruptures or lateral ankle strains
Orthèse pour le traitement de ruptures de ligaments ou de claquages pour cheville latérale

(30) Priorität: 03.08.1989 CH 2876/89
(43) Veröffentlichungstag der Anmeldung: 06.02.1991
(73) Patentinhaber: Amrein, Max, CH-1020 Renens (CH)
(72) Erfinder: Amrein, Max, CH-1020 Renens (CH)
(74) Vertreter: Feldmann, Clarence Paul

(56) Entgegenhaltungen:
- US-A- 3 613 674
- US-A- 4 724 847
- US-A- 4 825 856

## Beschreibung

In der Orthopädietechnik sind Orthesen zur Behandlung von Bänderverletzungen am lateralen Knöchel seit langem bekannt. Dabei hat man sich jedoch vorwiegend mit der Fixation befasst, was einer statischen Behandlung entspricht. Meist geschah dies durch Eingipsen oder mit relativ starren Orthesen, bei denen das Einschienen im Vordergrund stand. Bekanntlich führt jedoch die statische Behandlung zu einer Verlängerung der Behandlungszeit und bedingt eine relativ lange und intensive Nachbehandlung mit Massagen und Bewegungstherapien.

Eine Orthese zur dynamischen Behandlung von Bänderverletzungen, gemäß dem Oberbegriff des Anspruchs 1 ist aus der US-A-4 724 847 bekannt. Sie konnte jedoch nicht allen Anforderungen gerecht werden.

Es ist folglich die Aufgabe der vorliegenden Erfindung eine Orthese zu schaffen, die besonders zur dynamischen Behandlung von Bänderverletzungen am lateralen Knöchel geeignet ist, wobei insbesondere eine Eversion des Rückfusses gewährleistet und gegebenenfalls die Plantarflexion des Fusses verhütet werden kann.
Diese Aufgabe löst eine Orthese mit den Merkmalen des Patentanspruches 1. Weitere vorteilhafte Ausgestaltungsformen gehen aus den abhängigen Patentansprüchen hervor, und deren Wirkungsweise ist in der nachfolgenden Beschreibung erläutert.
In der beiliegenden Zeichnung ist ein bevorzugtes Ausführungsbeispiel dargestellt und in der nachfolgenden Beschreibung detailiert erklärt.

Es zeigt:
- Figur 1-5: die Einzelteile der Orthese;
- Figur 6: die Anordnung des Eversionskeiles an einem zu behandelnden Fuss;
- Figur 7: die Orthese von der medialen Seite gesehen in der Gebrauchsanordnung ohne Antiplantarflexionsband;

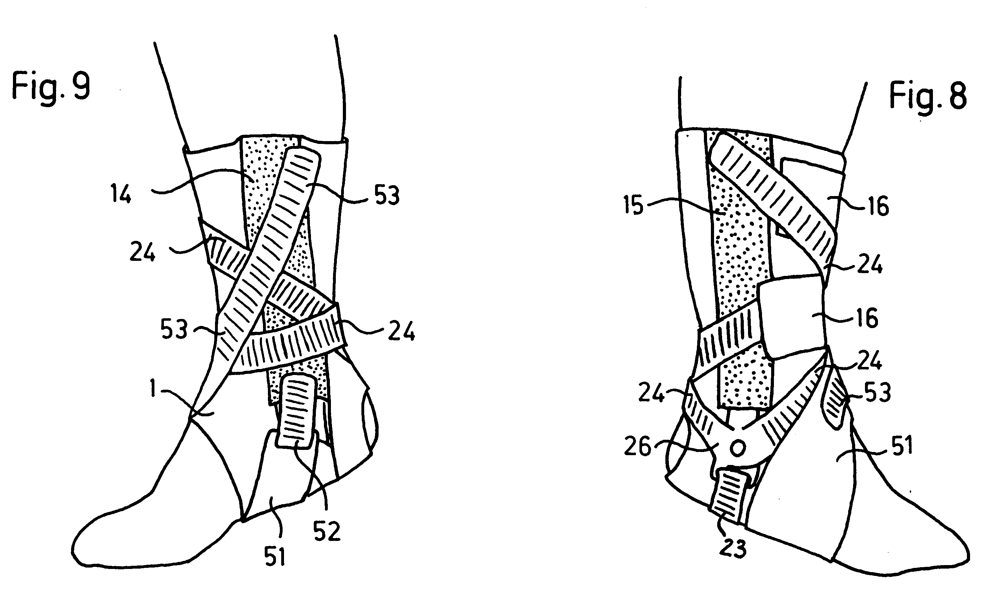

- Figur 8: Dieselbe Anordnung von der lateralen Seite gesehen mit einem Antiplantarflexionsband sowie
- Figur 9: die Anordnung nach Figur 8 von der medialen Seite gesehen.

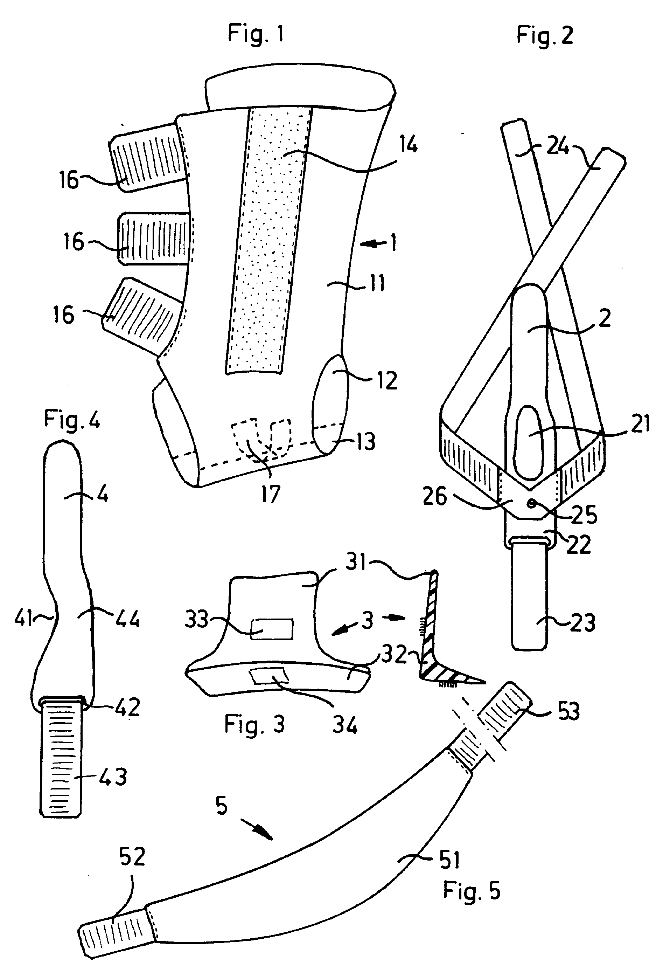

Vorerst seien die einzelnen Teile der Orthese ohne Bezug der Funktion beschrieben und danach deren funktionelle Bedeutung erläutert.
In der nachfolgenden Beschreibung werden die Begriffe medial und lateral häufig verwendet. Medial bedeutet auf der Innenseite, lateral auf der Aussenseite des Fusses gelegen. In Bezug auf die einzelnen Elemente der Orthese sind diese Begriffe selbstverständlich in Bezug auf den mit der Orthese geschützten Fuss bezogen.
Ferner wird mehrmals der Ausdruck Eversion verwendet. Bei einer Fussverstauchung wird der Fuss nach innen geknickt, wobei die äusseren Fussknöchelbänder, das heisst die lateralen Bänder, beschädigt werden können. Diese Bewegung nennt sich Inversion. Der geschädigte Fuss neigt somit ständig zur Inversion. Die entgegengerichtete Bewegung nennt sich Eversion.
Die ebenfalls nach einer Bänderverletzung vorkommende Neigung des Vorderfusses hinunter zu hängen, nennt sich Plantarflexion.
Das Grundelement der Orthese ist die in der Figur 1 dargestellte Knöchelbandage 1. Dieses wird aus drei massgeschneiderten und zusammengenähten Lappen zusammengesetzt, nämlich einem Innen- 11, einem Aussen- 12 und einem die beiden verbindenden Sohlenlappen 13. Die so geformte, unten geschlosssene Gamasche wird aus einem elastischen, doppelschichtigen gewirkten Textil mit einer dazwischen eingelagerten Polsterschicht gefertigt und ist für die erforderliche Luftzirkulation mit einer Perforation versehen. Auf der Aussenseite der Bandage sind medial und lateral je ein in Längsrichtung des Schaftteiles der Bandage 1 verlaufendes Klettband mit der nach aussen gerichteten Haftseite aufgenäht. Entsprechend ihrer Funktion seien diese als Schienenaufnahmeband 14, auf der medialen Seite (hier Sichtseite), respektive 15 auf der (hier nicht sichtbar) lateralen Seite, bezeichnet. Die Schienenaufnahmebänder sind mit der Bandage 1 vernäht und oben bündig mit der Oberkante der Bandage zugenäht An dem, in der Gebrauchslage medial am Unterschenkel anliegenden Innenlappen 11 sind drei elastische Klettbänder befestigt, die als Schliessbänder 16 der Bandage dienen. Entsprechende Gegenbänder sind am Aussenlappen 12 aussen aufgenäht. Strichliniert ist auf der Aussenseite der Bandage 1 am Aussenlappen 12 und am Sohlenlappen 13 ein der Haftung dienendes Gegenband, das als Positierungsband 17 wirkt, eingenäht. Dieses reicht etwa vom Bereich unterhalb des zu bandagierenden Knöchels bis cirka zur Mitte des Sohlenbereiches.
In das Schienenaufnahmeband 14 lässt sich die in Figur 2 dargestellte laterale Schiene 2, die im Knöchelbereich geringfügig verbreitet und dort eine der Knöchelform angepasste Ausnehmung 21 hat, einschieben.
Am unteren Ende ist ein Querschlitz 22 vorhanden, durch den ein Klettband geschlauft ist, welches als Schienenfixierband 23 dient. Es wirkt zusammen mit einem ebensolchen Gegenband 43, welches an der medialen Schiene 4 (Figur 4) befestigt ist. Jene Schiene 4 hat eine Einbuchtung 41 im Knöchelbereich und ist dort zudem mit einer der Knöchelform entsprechenden Wölbung 44 versehen. Das Gegenband 43 ist wiederum durch einen Querschlitz 42 im unteren Randbereich der Schiene 4 geschlauft.
In der Bandage 1 auf das Positionierungsband 17 lässt sich ein Eversionskeil 3 anbringen. Dieser Eversionskeit, der vorzugsweise aus geschäumtem Material , beispielsweise ein Polyurethanschaum hergestellt ist, hat zwei, einen etwa rechten Winkel, einschliessende Wände 31 und 32. Während die vertikale Wand 31 eine über die gesamte Fläche etwa gleichbleibende Dicke aufweist, ist die horizontale, plantare Wand keilförmig verjüngt. Beide Wände haben auf ihrer Aussen-, beziehungsweise Unterseite je einen Klettbandabschnitt 33, 34, die mit dem bereits beschriebenen Positionierungsband 17 zusammenwirken.
Letztlich zeigt Figur 5 noch ein Antiplantarflexionsband 5. Dieses besteht aus einem sichelbogenförmig geschnittenen Streifen 51, der beispielsweise aus Kunststoff, Kunstleder oder Leder gefertigt und mit einer Polsterschicht versehen ist. An den Enden ist je ein elastisches Klettband angenäht. Während das mediale Klettband 52 relativ kurz ist, ist das laterale Band 53 um ein Mehrfaches länger.

Das Antiplantarflexionsband hat einen antisupinatorischen Effekt, das heisst, eine gegen die Inversion gerichtete Wirkung. Daher konnte dieses Band auch Antisupinationsband genannt werden.

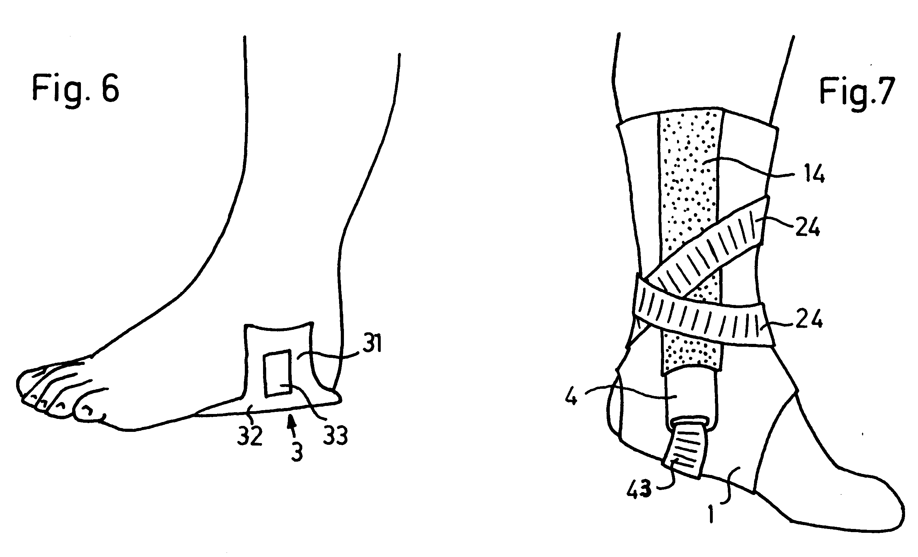

Bei der erstmaligen Verwendung der Orthese wird zuerst der Eversionskeil 3 angepasst, wie dies Figur 6 zeigt. Hierbei wird die vertikale Wand 31 falls erforderlich, bis unterhalb des lateralen Knöchels eingekürzt und die plantare keilförmige Wand 32 so angepasst, dass sie bis zur Mitte der Fusssohle auf Null sich verjüngt. Danach wird der Eversionskeil 3 mit seinen Klettbandabschnitten 33 und 34 in die Bandage 1 eingelegt und angedrückt, so dass der Klettbandabschnitt 33 am Positionierband 17 am Aussenlappen 12 und der Klettbandabschnitt 34 am Positionierband 17 am Sohlenlappen 13 haftet.
Zur Verminderung der Emboliegefahr wird ein elastischer Stützsocken angezogen und die Bandage 1 angelegt. Wegen der hohen Elastizität der Bandage kommt man mit nur drei verschiedenen Grössen für erwachsene Patienten aus. Mit den Schliessbändern 16 wird die Bandage 1 straff geschlossen. Danach schiebt man die mediale und laterale Schiene 2, beziehungsweise 4, in die entsprechenden Schienenaufnahmebänder 14, beziehungsweise 15, stellt ihre korrekte Lage fest und fixiert dieselben, bezüglich einander, mittels den entsprechenden Bändern 23 und 43. Die Relativlage der Schiene 2, bezüglich der Bandage 1, wird gesichert durch relativ lange Haltebänder 24, wiederum als elastische Klettbänder geformt. Die Bänder halten an ein Zwischenstück 26, welches mit einer Niet 25 an der lateralen Schiene 2 befestigt ist. Die langen Bänder 24 verlaufen spiralförmig um die Bandage 1 herum und kreuzen dabei, die als Gegenbänder ausgebildeten und als Schienenaufnahme dienenden Bänder 14 und 15, an denen sie haften. In dieser Lage wie in Figur 7 dargestellt ist, der Fuss durch die Schienen 2 und 4 gestützt und durch den Eversionskeil in eine leichte Eversionslage gehalten und so gegen eine Inversion geschützt. Die Festigkeit der Bandage 1 ergibt auch eine geringe Sicherheit bezüglich einer Plantarflexion. Im frühen Heilstadium wird man dies jedoch zusätzlich durch das Antiplantarflexionsband 5 sichern. Dessen kurzes Klettband 52 wird medial am Schienenaufnahmeband 14 befestigt und der sichelbogenförmige Streifen 51 unter dem Fuss durch, von medialer Seite zur lateralen Seite gelegt, und mit dem langen lateralen Band 53 über den Fuss hinweg wieder auf die mediale Seite hinüber gezogen, wodurch auch die gewünschte Eversion noch erhöht wird.
Vielfach neigen Patienten mit bereits einmal beschädigten Knöchelbänder wiederholt zu Verstauchungen. Für solche Fälle wird der Patient insbesondere beim Ausüben von Sport die Orthese auch lange nach der Ausheilung seiner Verletzung noch tragen. Dann kann und wird er jedoch häufig auf das Antiplantarflexionsband 5 verzichten. Jedoch darf der Eversionskeil nicht weggelassen werden.

## Patentansprüche

1. Orthese zur dynamischen Behandlung von Bänderrissen oder -zerrungen am lateralen Fussknöchel, bestehend aus einem textilen Innen-, Aussen- und Sohlenlappen (11, 12, 13), die mittels Schliessbändern (16) zu einer gamaschenartigen Bandage verschliessbar ist, dadurch gekennzeichnet, dass die Bandage (1) sowohl lateral als auch medial ein Schienenaufnahmeband (14, 15) aufweist, in denen je eine entsprechend geformte, laterale beziehungsweise mediale Schiene (2, 4) einschiebbar ist, an der ein nach Massgabe der Fussform örtlich variabel fixierbarer (33, 34), lateral angeordneter Eversionskeil (3) angebracht ist, der aus zwei mindestens annähernd senkrecht zu einander stehenden Wänden (31, 32) besteht, von denen die plantar angeordnete Wand (32) keilförmig von der lateralen Wand (31) zur Mitte des Sohlenlappens (13) hin abnimmt.

2. Orthese nach Anspruch 1, dadurch gekennzeichnet, dass sie ein Antiplantarflexionsband (5) umfasst, welches mit einem relativ kurzen, medialen Klettband (52) am medialen Schienenaufnahmeband (14) haftet und in der Form eines sichelförmig geschnittenen Streifens (51) unter dem Sohlenlappen (13) hindurch geführt ist und von der lateralen Seite her mit einem relativ langen, lateralen Klettband (53) zur medialen Seite auf demselben Schienenaufnahmeband (14) geführt ist, auf dem es haftet.

3. Orthese nach Anspruch 1, dadurch gekennzeichnet, dass die mediale Schiene (4) nur über ein Schienenfixierband (43) relativ zur lateralen Schiene (2) an dessen Schienenfixierband (23) gehalten ist.

4. Orthese nach Anspruch 3, dadurch gekennzeichnet, dass die laterale Schiene (2) mittels zweier relativ langer, elastischer Haltebänder (24), die an einem, an der Schiene (2) befestigten Zwischenstück gehalten sind bezüglich der Bandage (1) fixierbar ist.

5. Orhtese nach Anspruch 1, dadurch gekennzeichnet, dass die laterale Schiene (2) eine für den Knöchelbereich des zu stützenden Fusses gewünschte Ausnehmung (21) aufweist.

6. Orthese nach Anspruch 1, dadurch geknnezeichnet, dass die mediale Schiene (4) eine für den Knöchelbereich, des zu stützenden Fusses, gewünschte Wölbung (44) und Einbuchtung (41) aufweist.

7. Orthese nach Anspruch 1, dadurch gekennzeichnet, dass zur Fixierung des Eversionskeiles (3), dieser mit Klettbandabschnitten (33, 34) versehen ist und die Bandage (1) an der Innenfläche im Bereich des Sohlen- und Aussenlappens (12, 13) ein Positionierungsband (17) aufweist, auf dem die Klettbandabschnitte (33, 34) haften.

## Claims

1. Orthopaedic truss for the dynamic treatment of torn or strained ligaments of the lateral ankle, consisting of a textile inner, outer and sole flap (11,12,13) that can be closed by means of straps (16) to form a gaiter-like truss, characterised in that the truss (1) has both a lateral and a medial splint-retaining sheath (14,15) in which a matching, lateral or medial splint (2,4) can be inserted, to which splint a laterally disposed eversion wedge (3) is attached, the wedge being fixable (33,34) in variable positions according to the shape of the foot and consisting of two walls (31,32) forming at least approximately a right angle to each other, the wall (32) positioned under the sole forming a wedge shape growing thinner from the lateral wall (31) towards the centre of the sole flap (13).

2. Orthopaedic truss according to Claim 1, characterised in that it includes a strap (5) for resisting plantar flexure, which strap is attached to the medial splint-retaining sheath (14) by a relatively short, medial hook-and-loop fastening strip (52), is cut to form a crescent shaped strip (51) passing under the sole flap (13) and has a relatively long, lateral hook-and-loop fastening strip (53) passing from the lateral side to the same splint-retaining sheath (14) on the medial side to which it is fixed by its other end.

3. Orthopaedic truss according to Claim 1, characterised in that the medial splint (4) is held in relation to the lateral splint (2) only by a splint-fixing strap (43) attached to the fixing strap (23) of the lateral splint (2).

4. Orthopaedic truss according to Claim 3, characterised in that the lateral splint (2) is fixable in relation to the truss (1) by means of two relatively long elastic retaining straps (24) attached to an intermediate part [26? - tr.] mounted to the splint (2).

5. Orthopaedic truss according to Claim 1, characterised in that the lateral splint (2) has an opening (21) as desired for the ankle area of the foot to be supported.

6. Orthopaedic truss according to Claim 1, characterised in that the medial splint (4) has a convexity (44) and a recess (41) as desired for the ankle area of the foot to be supported.

7. Orthopaedic truss according to Claim 1, characterised in that to fix the eversion wedge (3) this wedge is provided with sections of hook-and-loop fastener (33, 34) and the inner surface of the truss (1) in the region of the sole and outer flaps (12,13) is provided with a positioning strip (17) to which the fastening strips (33, 34) adhere.

## Revendications

1. Orthèse destinée au traitement dynamique de déchirures ou d'arrachements de ligaments sur le malléole latéral, se composant d'une pièce d'étoffe textile intérieure, extérieure et de semelle (11, 12, 13), qui peut être fermée au moyen de bandes de fermeture (16) selon un bandage en forme de guêtre, caractérisée en ce que le bandage (11) présente, tant sur les côtés qu'au milieu, des bandes réceptrices (14, 15) de tiges, dans chacune desquelles est insérable une tige latérale ou médiane (2, 4) de forme appropriée, sur laquelle est montée une cale de renversement (3) disposée latéralement, apte à être fixée localement (33, 34) de manière variable selon les données de la forme du pied et qui se compose d'au moins deux parois (31, 32) disposées à peu près perpendiculairement l'une à l'autre, la paroi (32) disposée sous la plante du pied se réduisant en forme de cale depuis la paroi latérale (31) jusqu'au milieu de la pièce d'étoffe formant la semelle (13).

2. Orthèse selon la revendication 1, caractérisée en ce qu'elle comprend une bande de flexion antiplantaire (5) qui adhère au moyen d'une bande collante médiane relativement courte (52) sur la bande réceptrice médiane (14) de tige et qui est guidée sous forme d'une bande découpée en lame de faux (51) au-dessous de la bande de semelle (13) et à partir du côté latéral au moyen d'une bande latérale relativement longue (53), collant sur le côté médian sur la même bande réceptrice (14) de tige, à laquelle elle adhère.

3. Orthèse selon la revendication 1, caractérisée en ce que la tige médiane (4) n'est maintenue, vis-à-vis de la tige latérale (2), que par une bande de fixation (43), sur la bande de fixation (23) de cette tige latérale.

4. Orthèse selon la revendication 3, caractérisée en ce que la tige latérale (2) peut être fixée vis-à-vis du bandage (1) au moyen de deux bandes de maintien élastiques relativement longues (24), maintenues sur un élément intermédiaire fixé sur la tige (2).

5. Orthèse selon la revendication 1, caractérisée en ce que la tige latérale (2) présente un évidement (21), prévu pour la zone de malléole du pied à supporter.

6. Orthèse selon la revendication 1, caractérisée en ce que la tige médiane (4) présente un bourrelet (44), prévu pour la zone de malléole du pied à supporter, ainsi qu'un retrait (41) en forme d'anse.

7. Orthèse selon la revendication 1, caractérisée en ce que la cale de renversement (3) est pourvue, pour sa fixation, de parties de bandes collantes (33, 34) et que le bandage (1) présente dans ce même but, sur la surface intérieure dans la zone de la pièce d'étoffe de semelle et extérieure (12,13), une bande de positionnement (17) sur laquelle adhèrent les parties de bandes collantes (33, 34).
